# DEMANDE DE BREVET EUROPEEN

(11) **EP 1 891 994 A1**
(43) Date de publication de la demande: **27.02.2008**
(21) Numéro de dépôt: 07290984.9
(22) Date de dépôt: 07.08.2007
(51) Int. Cl.: A61L 31/10, A61F 2/06

(54) **Endoprothèse et système comprenant une telle endoprothèse**

(30) Priorité: 07.08.2006 FR 0607204
(71) Demandeur: Bergeron, Patrice, 83330 Le Beausset (FR)
(72) Inventeur: Bergeron, Patrice, 83330 Le Beausset (FR)
(74) Mandataire: Bredema

(57) **Abrégé**

La présente invention concerne un endoprothèse tubulaire constituée par une armature de forme sensiblement tubulaire revêtue par une membrane d'étanchéité, présentant au moins une extrémité biseautée, caractérisée en ce qu'elle est constituée par une armature en un matériau auto-expansible revêtue par une membrane en PTFE ou en Dacron.

## Description

La présente invention concerne le domaine des endoprothèses pour le traitement d'anévrismes vasculaires.

On connaît dans l'état de la technique le brevet européen EP1014890 décrivant un ensemble pour le traitement des anévrismes et des dissections vasculaires constitué par un ballonnet d'expansion et par une endoprothèse formée par une armature expansible recouverte par un matériau d'étanchéité et présentant une forme cylindrique dont l'extrémité biseautée réalise un plan frontal, caractérisé en ce que le ballonnet présente une forme générale cylindrique prolongée à l'une de ses extrémités par une partie évasée.

Le but de la présente invention est de proposer une amélioration aux endoprothèses biseautées pour le traitement de certaines pathologies et en particuliers des anévrismes et des dissections de l'aorte abdominale et thoracique, en particulier affectant la crosse aortique.

À cet effet, l'invention concerne selon son acception la plus générale une endoprothèse tubulaire constituée par une armature de forme sensiblement tubulaire revêtue par une membrane d'étanchéité, présentant au moins une extrémité biseautée, caractérisée en ce qu'elle est constituée par une armature en un matériau auto-expansible revêtue par une membrane en PTFE ou en Dacron.

De préférence, l'armature auto-expansible est réalisée par des fils de Nitinol.

Avantageusement, l'armature auto-expansible est encapsulée entre deux membranes de PTFE.

Selon une variante, le diamètre est constant et compris entre 26 et 46 millimètres.

Selon un mode de réalisation particulier, elle présente une forme conique.

De préférence, la longueur comprise entre 10 et 30 cm.

Selon un mode de réalisation particulier, l'endoprothèse présente une forme arquée.

L'invention concerne également un système comprenant une endoprothèse tubulaire constituée par une armature de forme sensiblement tubulaire revêtue par une membrane d'étanchéité, présentant au moins une extrémité biseautée, un cathéter de largage par retrait de la gaine externe.

L'invention sera mieux comprise à la lecture de la description d'exemples non limitatifs, où :
- la figure 1 représente une vue d'un premier exemple de réalisation ;
- la figure 2 représente une vue d'une variante de réalisation conique ;
- les figures 3 et 4 représentent l'application des endoprothèses biseautées à la crosse aortique (justifiée par les différences de longueurs entre les courbures inférieure et supérieure).

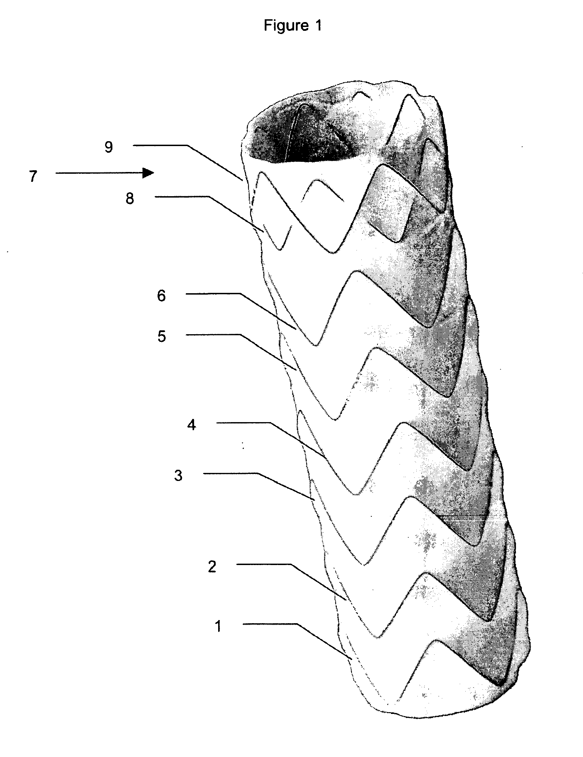

La figure 1 représente une vue d'un premier exemple de réalisation de configuration tubulaire. L'endoprothèse est constituée par une armature formée par une succession de couronnes (1 à 6). Chaque couronne est constituée par un fil en un matériau à mémoire de forme. A titre d'exemple, le matériau est alliage à base de nickel et de titane dénommé Nitinol (nom commercial) qui a la particularité de changer d'état en fonction de la température. En deçà d'une température de transition correspondant sensiblement à la température du corps humain, soit de l'ordre de 36°, sa structure cristalline est dite martensitique. Au-delà, elle est austénitique. Lorsque l'on soumet cet alliage dans sa phase à haute température à des contraintes mécaniques, comme un allongement forcé, puis qu'on le refroidit, il garde en mémoire ces contraintes. Dès qu'on le chauffe à nouveau, il reprend sa forme obtenue sous contrainte.

Il est formé pour présenter une forme sous contrainte dont la section est inférieure à la section d'implantation. Il reprend automatiquement sa forme initiale, de section plus importante, après l'introduction dans le corps humain.

Le fil forme un anneau continu présentant des ondulations longitudinales. Le rayon de courbure des changements de direction est d'environ 90°, et l'amplitude des ondulations est comprise entre 10 et 30 millimètres.

Les couronnes (1 à 6) sont espacées longitudinalement d'une distance correspondant sensiblement à l'amplitude des ondulations. Deux couronnes consécutives ne présentent aucune intersection.

Le diamètre du fil de Nitinol est d'environ 1 millimètre (souvent plus petit). L'une des extrémités non visible sur la figure 1 est biseautée. L'autre extrémité présente une couronne (7) formée de deux fils (8, 9) formant des anneaux avec des ondulations longitudinales se chevauchant (en principe, c'est cette extrémité-là qu'on peut être amené à biseauter : extrémité proximale). Ces deux anneaux sont décalés angulairement d'un demi-pas, afin que les ondulations se recoupent périodiquement.

Cette armature est revêtue, à l'intérieur et à l'extérieur, par une membrane de PTFE (polytétrafluoroéthylène) assurant l'étanchéité de l'endoprothèse, ainsi que le maintien longitudinal des couronnées en Nitinol.

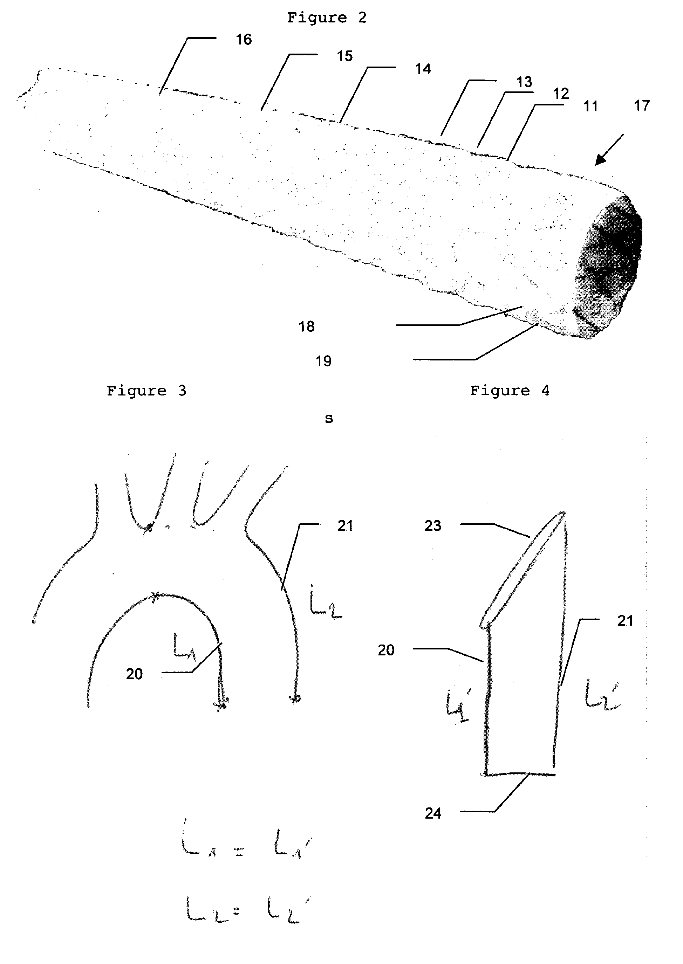

La figure 2 représente une variante de réalisation où les couronnes présentent des sections décroissantes, depuis la couronne (17) formée par recouvrement de deux fils (17, 18). Il s'agit de la présentation conique.

Les figures 3 et 4 représentent une variante de réalisation où l'endoprothèse présente une forme arquée. La longueur du segment intérieur (20) est inférieure à la longueur du segment opposé (21). L'extrémité biseautée (23) présente par ailleurs un diamètre supérieur à l'extrémité opposée (24).

L'endoprothèse est introduite dans l'organisme par endoscopie à partir d'une incision fémorale ou iliaque, et mis en place à l'endroit voulu. La chaleur corporelle le réchauffe, il retourne à sa phase austénitique pour laquelle il a la forme d'un treillis cylindrique qui vient se plaquer sur la paroi artérielle et la renforcer, formant un chemisage de l'artère malade.

Avantageusement une courte endoprothèse biseautée autoexpansible peut servir d'extension à une prothèse non biseautée déjà implantée pour améliorer la couverture de la zone malade (longueur entre 20 et 80 millimètres).

## Revendications

1. - Endoprothèse tubulaire constituée par une armature de forme sensiblement tubulaire revêtue par une membrane d'étanchéité, présentant au moins une extrémité biseautée, **caractérisée en ce qu'**elle est constituée par une armature en un matériau auto-expansible revêtue par une membrane en PTFE ou en Dacron.

2. - Endoprothèse selon la revendication 1, **caractérisée en ce que** l'armature auto-expansible est réalisée par des fils de nitinol.

3. - Endoprothèse selon la revendication 1 ou 2, **caractérisée en ce que** l'armature auto-expansible est encapsulée entre deux membranes de PTFE.

4. - Endoprothèse selon l'une au moins des revendications précédentes, **caractérisée en ce que** le diamètre est constant et compris entre 26 et 46 millimètres.

5. - Endoprothèse selon l'une au moins des revendications précédentes, **caractérisée en ce qu'**elle présente une forme conique.

6. - Endoprothèse selon l'une au moins des revendications précédentes, **caractérisée en ce qu'**elle présente une longueur comprise entre 10 et 30 cm.

7. - Endoprothèse selon l'une au moins des revendications précédentes, **caractérisée en ce qu'**elle présente une forme arquée.

8. - Système comprenant une endoprothèse tubulaire conforme à la revendication 1, constituée par une armature de forme sensiblement tubulaire réalisée en un matériau autoexpansible revêtue par une membrane d'étanchéité en PTFE ou en Ducron, présentant au moins une extrémité biseautée, le système comprenant en outre un cathéter de largage par retrait de la gaine externe.
